# FASCICULE DE BREVET EUROPEEN

(11) **EP 3 242 695 B1**
(45) Date de publication et mention de la délivrance du brevet: **26.09.2018**
(21) Numéro de dépôt: 16701982.7
(22) Date de dépôt: 06.01.2016
(51) Int. Cl.: A61M 5/00, A61B 17/3209, A61B 17/34

(54) **DISPOSITIF POUR ACTE MÉDICAL COMPRENANT D'UN CÔTÉ UN TROCART ET DE L'AUTRE CÔTÉ UNE CANULE**
VORRICHTUNG FÜR EIN MEDIZINISCHES VERFAHREN MIT EINEM TROKAR AN EINEM ENDE UND EINER KANÜLE AM ANDEREN ENDE
DEVICE FOR A MEDICAL PROCEDURE, COMPRISING A TROCAR AT ONE END AND A CANNULA AT THE OTHER END

(30) Priorité: 06.01.2015 FR 1550072
(43) Date de publication de la demande: 15.11.2017
(73) Titulaire: Needle Concept, 64200 Biarritz (FR)
(72) Inventeur: BUCHS, Olivier, 40300 Peyrehorade (FR); DJIANE, Grégory, 95210 Saint Gratien (FR)
(74) Mandataire: Gaillarde, Frédéric F. Ch.
(86) Numéro de dépôt international: PCT/FR2016/050019
(87) Numéro de publication internationale: WO 2016/110649

(56) Documents cités:
- WO-A1-91/08787
- WO-A2-2005/044116

## Description

La présente invention est définie par la revendication 1 et concerne un dispositif supportant à la fois une aiguille rigide formant un trocart ainsi qu'une aiguille généralement souple formant une canule, prévu pour réaliser des opérations chirurgicales.

Un dispositif de l'art antérieur est décrit dans le document WO2005/044116 A2. Pour réaliser certaines opérations chirurgicales comme des injections, des ponctions ou des biopsies, il est connu d'utiliser un instrument appelé trocart, comportant un manche prolongé par une tige pointue permettant de percer la peau des patients.

On installe ensuite dans le perçage une canule formée par un tube généralement relativement souple comprenant à son extrémité avant un orifice inséré dans le corps à l'endroit voulu, et à son extrémité arrière un embout permettant de le relier notamment à une seringue ou à une poche pour des injections ou des perfusions, ou à un moyen effectuant des prélèvements.

L'opérateur utilise dans ce cas deux outils distincts pour effectuer successivement ces deux opérations. Il faut alors préparer et stériliser de manière indépendante ces deux outils, puis les emballer et les conditionner pour les expédier aux centres de soins. Ces opérations séparées entraînent des coûts relativement importants, et donnent par ces emballages distincts un encombrement élevé qui occupe de la place et augmente les frais.

De plus dans le centre de soins il faut approvisionner au poste de travail les deux outils, que l'opérateur devra successivement déballer de son emballage stérile afin de s'en servir chacun à son tour. Ensuite après la première opération de perçage avec le trocart il devra le reposer, puis saisir la canule pour l'insérer dans le corps, ce qui fait des manipulations successives.

En particulier après la repose du trocart présentant son aiguille à l'air libre, il y a un risque d'incident par piqûre avec cette aiguille lors des manipulations suivantes, par l'opérateur ou par du personnel d'accompagnement, notamment lors de l'évacuation des outils après l'opération.

La présente invention a notamment pour but d'éviter ces inconvénients de la technique antérieure.

Elle propose à cet effet un dispositif pour acte médical, comprenant un manche supportant axialement d'un côté une aiguille rigide formant un trocart prévu pour percer la peau, le dispositif étant équipé d'un bouchon d'étanchéité recouvrant au moins l'aiguille rigide, remarquable en ce qu'il comporte de l'autre côté un tube protecteur allongé monté sur ce manche, contenant une aiguille formant une canule, c'est-à-dire remarquable en ce que le dispositif comporte un tube protecteur allongé monté sur le manche de l'autre côté dudit manche, ledit tube protecteur allongé contenant une aiguille formant une canule.

Un avantage de ce dispositif est qu'en prévoyant de chaque côté de manière protégée l'aiguille rigide formant trocart ainsi que l'aiguille de préférence souple formant canule, on réalise un sous-ensemble comprenant les outils pour les deux opérations successives, nécessitant une seule opération de stérilisation et recevant un emballage compact, ce qui facilite les manipulations et réduit les frais.

De plus la préparation du poste de travail est facilitée par l'approvisionnement d'un unique dispositif, qui permet après la première opération de perçage de la peau de remettre rapidement en place le bouchon protégeant l'aiguille rigide ce qui la protège, pour ensuite en retournant le manche sortir la canule de son tube protecteur afin de l'utiliser pour l'opération suivante d'injection ou de prélèvement.

Le dispositif pour acte médical, par exemple une opération chirurgicale, selon l'invention peut comporter de plus une ou plusieurs des caractéristiques suivantes, qui peuvent être combinées entre elles.

Avantageusement le dispositif comprend un capuchon de protection monté ou fixé sur le manche, le capuchon de protection étant recouvert par le bouchon et recouvrant l'aiguille rigide.

Le manche peut comporter une partie avant sensiblement cylindrique contenant un perçage axial recevant l'extrémité arrière de l'aiguille rigide, qui reçoit et maintient par serrage le capuchon.

Avantageusement, le capuchon comporte à l'extrémité avant un bourrelet circulaire extérieur, présentant une face avant plane. Le bourrelet facilite une traction sur le capuchon pour l'extraire, et de plus élargit la face plane donnant une meilleure stabilité à ce capuchon posé debout.

Avantageusement, le bouchon recouvre le capuchon et le manche, et présente une partie sensiblement cylindrique serrant le tube protecteur. On réalise ainsi un maintien de la stérilisation de l'ensemble du dispositif intérieur au moyen d'un unique serrage qui doit être étanche pour conserver la stérilité à l'intérieur.

Dans ce cas, avantageusement les surfaces de serrage du tube protecteur et du bouchon l'une sur l'autre, sont formées par un moulage en matière plastique dans une partie de moule circulaire continue donnant une absence de bavure. Cette absence de bavure évite des passages pouvant laisser entrer des germes d'infection.

Avantageusement, le tube protecteur et le bouchon comportent des nervures ou des stries axiales extérieures. Ces nervures et ces stries facilitent la prise en main des composants, ainsi qu'une rotation et une traction permettant d'ouvrir le bouchon.

En particulier le tube protecteur peut comporter quatre nervures disposées en croix, qui s'étendent sensiblement sur toute la longueur du tube contenant la canule. Ces nervures apportent avec une quantité de matière réduite un renforcement important du tube.

Avantageusement, le dispositif comporte des nervures axiales assurant sur leurs contours extérieurs le serrage d'un porte-canule dans le tube protecteur, du tube protecteur dans le manche, ou du manche dans le capuchon. On limite ainsi le serrage des deux éléments, facilitant le démontage.

Avantageusement, le dispositif comporte un porte-canule présentant vers l'avant un filetage extérieur comprenant au moins deux pas différents se superposant. On peut ainsi facilement fixer ce porte-canule sur différents modèles de seringues habituellement commercialisés.

Avantageusement, les différents composants moulés de ce dispositif sont formés par un moulage d'une matière plastique comprenant des formes permettant un démoulage axial.

L'invention sera mieux comprise et d'autres caractéristiques et avantages apparaîtront plus clairement à la lecture de la description ci-après donnée à titre d'exemple, en référence aux dessins annexés dans lesquels :
- les figures 1, 2 et 3 sont des vues respectivement de côté, en coupe axiale et en perspective, d'un dispositif complet pour opération chirurgicale selon l'invention ;
- les figures 4 à 8 sont des vues de ce dispositif présentant respectivement dans l'ordre des opérations, le bouchon retiré, le capuchon de l'aiguille rigide retiré, le capuchon remis, le manche du trocart retiré, et le tube protecteur de la canule retiré avec une seringue mise en place ;
- la figure 9 est une vue de côté d'un dispositif complet suivant une variante ; et
- les figures 10 et 11 sont des vues de ce dispositif présentant respectivement le bouchon retiré, puis le capuchon de l'aiguille rigide retiré.

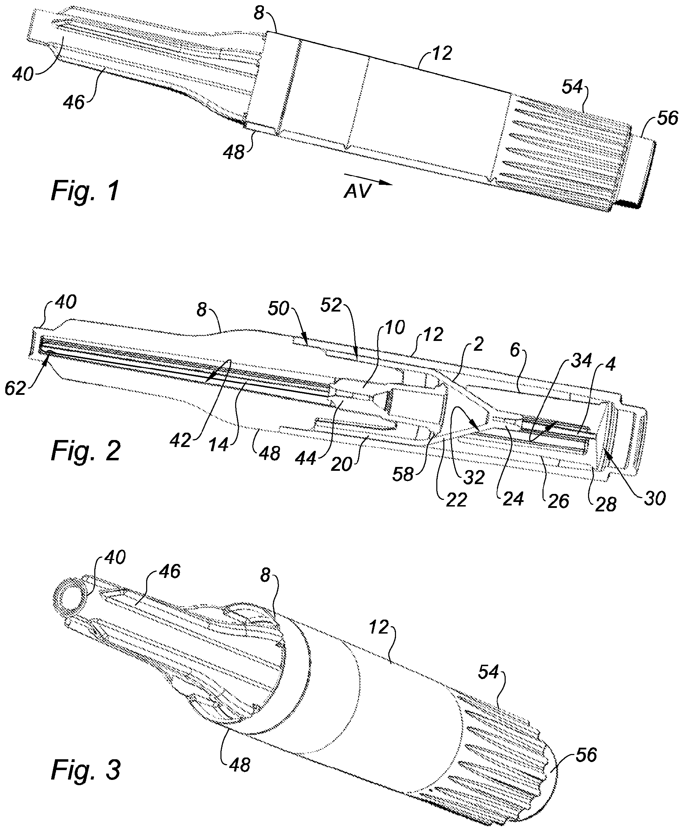

Les figures 1, 2 et 3 présentent un dispositif pour acte médical tel qu'une opération chirurgicale formant un ensemble allongé suivant un axe principal, comprenant un manche intérieur 2 supportant du côté avant indiqué par la flèche notée « AV », une aiguille rigide 4 formant un trocart prévu pour percer la peau d'un patient. Est entendu par le vocable « trocart » un ensemble comprenant un manche auquel est fixée une aiguille ou tige rigide et perçante.

L'aiguille rigide est protégée par un capuchon 6 venant se fixer autour du manche 2, cet ensemble est lui-même protégé par un bouchon 12 d'étanchéité venant aussi se fixer ici autour d'un tube protecteur 8.

Le manche 2 reçoit du côté arrière le tube protecteur 8 qui vient se fixer à l'intérieur. Le tube protecteur 8 reçoit du côté avant un porte-canule 10 serré dans ce tube, qui supporte une canule 14 s'étendant jusqu'à l'extrémité arrière de ce tube.

L'ensemble des composants du dispositif est réalisé par un moulage par injection d'une matière plastique, comportant avantageusement du polypropylène ou du polycarbonate. L'aiguille rigide 4 ainsi que la canule 14 sont réalisées en acier inoxydable.

Le manche 2 comporte une partie circulaire creuse sensiblement cylindrique 20, se prolongeant vers l'avant par une partie conique 22 se resserrant, qui se termine par une forme sensiblement cylindrique 24 recevant dans un perçage axial avant, l'extrémité arrière de l'aiguille rigide 4.

L'aiguille rigide 4 comporte une extrémité arrière prévue pour se serrer dans le perçage axial avant du manche 2, et une pointe avant qui peut comporter différentes formes couramment utilisées pour les trocarts, comprenant notamment une section transversale en croix se terminant par une pointe facilitant le perçage de la peau.

Le capuchon 6 comporte une forme extérieure sensiblement cylindrique 26, se terminant à l'extrémité avant par un bourrelet circulaire extérieur 28, présentant une face avant plane 30 comprenant un petit creux intérieur. Cette face avant plane 30 permet après avoir retiré le bouchon 12 de poser debout le dispositif, le bourrelet extérieur 28 assurant une assise plus large lui donnant plus de stabilité dans cette position ainsi qu'une meilleure accroche pour le capuchon 6 en position assemblée.

Le capuchon 6 comporte à l'intérieur en partant du côté arrière, un creux conique qui se resserre 32, puis un perçage sensiblement cylindrique 34 qui se prolonge presque jusqu'à l'extrémité, recevant l'aiguille rigide 4. Le perçage 34 comporte sur sa paroi intérieure des nervures axiales s'étendant sur toute la longueur, qui réduisent la quantité de matière.

La partie arrière du creux cylindrique 34 s'ajuste sur la forme cylindrique avant 24 du manche 2, pour se serrer dessus et maintenir le capuchon 6 en place lors de son assemblage, les nervures axiales de ce creux limitant le serrage. Le bourrelet extérieur du capuchon 28 constitue une forme permettant une traction dessus pour faciliter son retrait du manche 2.

Les formes sensiblement cylindriques du capuchon 6 comportent une petite pente donnant vers l'arrière un resserrement du diamètre de la forme cylindrique extérieure 26 et une augmentation du diamètre du perçage intérieur 34, constituant des angles de dépouille permettant un démoulage axial du capuchon à partir d'un moule comprenant des formes circulaires en une seule partie, qui se retirent en coulissant vers l'arrière.

Le tube protecteur 8 comporte en partant de l'arrière une forme extérieure sensiblement cylindrique 40 de petit diamètre, contenant à l'intérieur un perçage sensiblement cylindrique 42 débouchant vers l'avant, recevant la canule 14. Le perçage cylindrique 42 comporte sur sa paroi intérieure des petites nervures axiales s'étendant sur toute sa longueur, qui réduisent la quantité de matière.

Le porte-canule 10 se termine vers l'arrière par des nervures axiales 44 présentant des arrêtes formant un contour extérieur sensiblement cylindrique, recevant le serrage du perçage 42 du tube protecteur 8, qui est lisse dans cette partie. L'appui sur les arrêtes des nervures 44 permet de limiter le serrage, et facilite le desserrage.

Le porte-canule 10 se prolonge vers l'avant par une partie sensiblement cylindrique se terminant par un filetage extérieur 58 comprenant deux pas différents se superposant, de 1.25 et 2.5mm, permettant le vissage d'une seringue du commerce qui présente habituellement l'un de ces deux pas.

Un perçage axial avant du porte-canule 10 reçoit la canule 14 formée par une aiguille creuse recevant le liquide délivré par la seringue, présentant une extrémité avant arrondie comportant un trou d'injection sur le côté 62, de manière à ne pas piquer la peau ou les chairs lors de son introduction dans le perçage réalisé auparavant par l'aiguille rigide 4.

La forme extérieure cylindrique 40 du tube protecteur 8 est renforcée un peu après l'extrémité arrière par quatre nervures 46 disposées en croix dans une section transversale, comprenant d'abord une hauteur sensiblement constante, qui vers le milieu de ce tube présente un fort agrandissement suivant une courbe régulière. Les arrêtes extérieures des nervures 46 arrivent de manière tangente sur la surface extérieure d'une couronne creuse cylindrique 48, ces nervures se prolongeant à l'intérieur de cette couronne pour la soutenir.

Les quatre nervures en croix 46 permettent de renforcer efficacement avec une masse de matière réduite, la forme cylindrique 40 constituant le tube de protection de la canule 14 insérée dedans. Ces nervures 46 permettent également de faciliter la préhension du tube protecteur 8 avec la canule 14 pour le vissage porte-canule 10 avec la seringue au moyen du filetage extérieur 58.

Ce vissage est également permis par les nervures axiales disposées sur la paroi intérieure du perçage cylindrique 42 du tube protecteur 8 qui sont en prise, en position assemblée, avec les nervures axiales 44 à l'arrière du porte-canule 10. Ces nervures axiales sont engrenées ensemble, en position assemblée, permettant la transmission d'efforts, et notamment la rotation du porte-canule 10, et donc du filetage extérieur 58, par la rotation du tube protecteur 8, laquelle est facilité par les nervures 46.

Par ailleurs, ces nervures axiales sont agencées en prise de sorte que, en cas de couple trop important, la transmission d'efforts n'est plus assurée. Cela est ici permis du fait des matériaux plastiques employés, et des déformations élastiques desdites nervures. Cela permet notamment de contrôler le couple de serrage entre le porte-canule 10 et la seringue. En effet, une fois le couple de serrage prédéterminé dépassé, la rotation du tube protecteur n'entraine plus celle du porte-canule, limitant ainsi le couple de serrage entre le porte-canule 10 et la seringue.

Le tube protecteur 8 comporte ensuite vers l'avant après la couronne creuse 48, un premier rétrécissement de diamètre formant une partie cylindrique mâle 50 recevant le serrage du bouchon 12, puis un deuxième rétrécissement comprenant des nervures axiales avant 52 présentant des arrêtes formant un contour extérieur sensiblement cylindrique, recevant le serrage du manche 2. L'appui sur les arrêtes des nervures avant 52 permet de limiter le serrage, et facilite le desserrage.

Les formes du tube protecteur 8 comportent pour la forme extérieure cylindrique 40 et la partie des arrêtes des nervures 46 de hauteur sensiblement constante, une petite pente donnant un resserrement vers l'arrière formant un angle de dépouille permettant un démoulage axial du moule dans ce sens.

Par ailleurs les formes du tube protecteur 8 comportent pour la partie cylindrique mâle 50 et l'arrête extérieure des nervures axiales avant 52 une petite pente donnant un resserrement vers l'avant, ainsi que pour le perçage intérieur 42 une petite pente donnant une augmentation vers l'avant, permettant un démoulage axial du moule dans ce sens.

Le bouchon 12 d'étanchéité comporte une forme globale sensiblement cylindrique d'épaisseur constante, comprenant vers l'arrière un diamètre intérieur venant s'ajuster sur la partie cylindrique mâle 50 du tube protecteur 8, afin d'obtenir un maintien efficace ainsi qu'une étanchéité maintenant la stérilité à l'intérieur.

Le bouchon 12 comporte vers l'arrière sur l'extérieur une série de stries axiales 54, facilitant la prise en main ainsi que l'adhérence pour tourner et tirer sur ce bouchon afin de le retirer du tube protecteur 8, l'autre main serrant ce tube par les nervures 46 facilitant aussi la préhension. Le bouchon 12 comporte ensuite une partie courte cylindrique de diamètre réduit 56, formant l'extrémité.

Le bouchon 12 comporte pour ses formes intérieure et extérieure une petite pente donnant un rétrécissement vers l'avant, permettant un démoulage axial du moule extérieur vers l'avant et du moule intérieur vers l'arrière.

Avantageusement la forme extérieure du tube protecteur 8 et la forme intérieure du bouchon 12 sont réalisées par un moule comprenant au niveau des surfaces de serrage de ces deux composants, dans la section transversale une forme circulaire continue, ce qui évite la production de petites bavures axiales lors du moulage qu'il est difficile d'éviter entre les deux parties d'un moule présentant un plan de joint suivant un plan axial. On obtient ainsi un bon serrage ainsi qu'un contact continu sur tout le pourtour de ces deux composants, ce qui assure l'étanchéité ainsi que le maintien de la stérilisation intérieure.

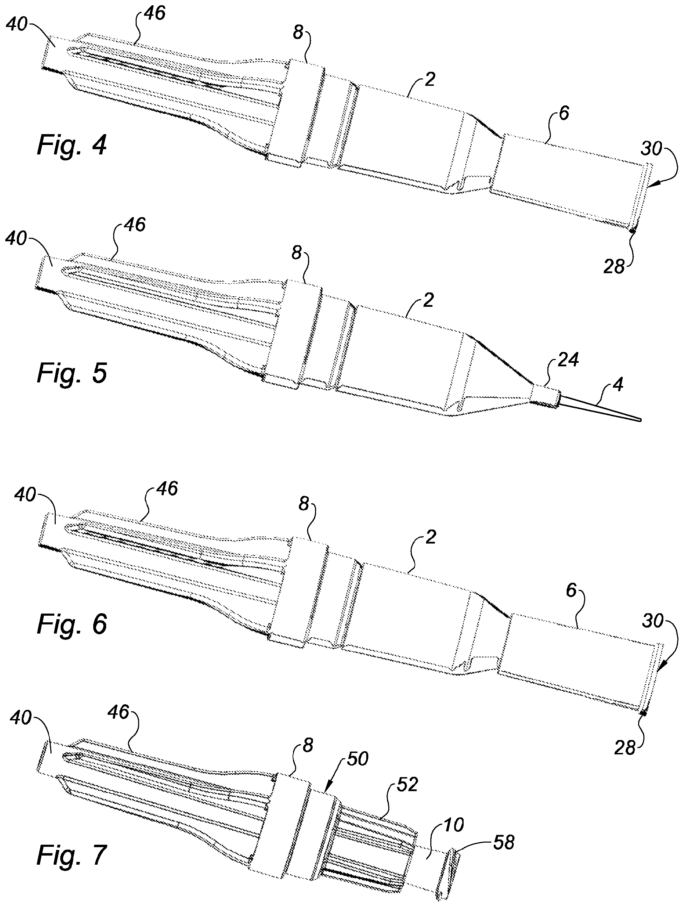

La figure 4 présente une première étape d'utilisation du dispositif pour acte médical qui a été sorti de son emballage, l'opérateur ayant retiré sur le lieu même de l'opération le bouchon 12 d'étanchéité en tournant et tirant sur sa partie comprenant les stries axiales 54, pour préparer cette opération.

On notera que le dispositif peut être disposé debout en le posant sur la face avant plane 30 du capuchon 6, qui étant large assure une bonne stabilité.

La figure 5 présente une étape suivante. L'opérateur ayant retiré le capuchon 6 en tirant sur son bourrelet extérieur avant 28, utilise le dispositif comme un crayon présentant une bonne prise en main grâce aux nervures 46 du tube protecteur 8, pour percer la peau avec l'aiguille rigide 4.

La figure 6 présente une étape suivante, où l'opérateur a remis le capuchon 6 sur l'aiguille rigide 4 pour assurer une protection de cette aiguille empêchant de se piquer avec lors des opérations suivantes comprenant l'élimination des outils. La face avant plane 30 du capuchon 6 permet alors de poser debout le capuchon,

La figure 7 présente une étape suivante où l'opérateur a retiré le manche 2 du tube protecteur 8, ce qui dégage le porte-canule 10 comprenant sa canule 14 fixée à son extrémité arrière.

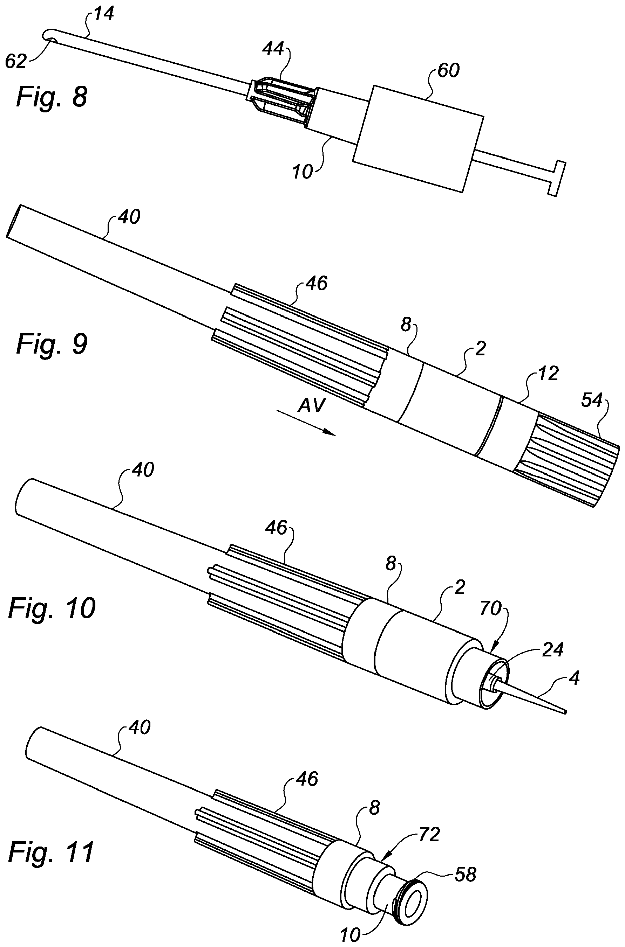

La figure 8 présente une étape suivante où l'opérateur a fixé une seringue 60 sur l'extrémité avant du porte-canule 10, puis retiré le tube protecteur 8 de ce porte-canule ce qui dégage la canule 14 qui devient opérationnelle.

On notera que lors de toutes les opérations l'opérateur garde à portée de main les deux outils comprenant le trocart formé par l'aiguille fixe 4 et la canule 14, qui restent toujours protégés quand ils ne sont pas utilisés.

Avantageusement on réalise lors de la fabrication du dispositif selon l'invention, après avoir assemblé l'ensemble de ses composants, une stérilisation du dispositif complet par l'oxyde d'éthylène qui a pour avantage de passer au travers des matières plastiques. L'assemblage préalable de ce dispositif ne gêne alors pas cette opération de stérilisation. D'autres procédés de stérilisation peuvent être également utilisés, tel qu'une stérilisation gamma avec des doses de rayons adaptées. Dans ce cas également la stérilisation peut être effectuée après avoir assemblé l'ensemble des composants du dispositif.

Les figures 9 à 11 présentent un dispositif suivant une variante comprenant sensiblement les mêmes composants, qui fonctionne de manière similaire.

Dans ce mode de réalisation, le dispositif comporte un bouchon 12 d'étanchéité mais ne comporte pas de capuchon 6. Le bouchon 12 assure également sensiblement les fonctions du capuchon et se termine à l'extrémité avant par un une face avant plane permettant de poser debout le bouchon avec l'aiguille rigide.

Le bouchon 12 comprenant les stries axiales extérieures 54 est court, il se monte ou fixe directement sur une partie cylindrique mâle avant 70 du manche 2. Le tube protecteur 8 comporte une partie cylindrique mâle tournée vers l'avant 72, recevant le serrage du manche 2. On notera que dans cette version le dispositif comporte deux serrages au lieu d'un pour la version précédente, qui doivent être étanches pour conserver la stérilité à l'intérieur.

Le tube protecteur 8 comporte en partant de l'arrière une partie extérieure cylindrique 40 qui est longue, les nervures extérieures 46 commençant seulement sensiblement au milieu de ce tube.

Le porte-canule 10 présente à son extrémité avant les deux filetages extérieurs 58 servant à la fixation sur la seringue.

## Revendications

1. Dispositif pour acte médical, comprenant un manche (2) supportant axialement d'un côté une aiguille rigide (4) formant un trocart prévu pour percer la peau, le dispositif étant équipé d'un bouchon (12) d'étanchéité recouvrant au moins l'aiguille rigide, **caractérisé en ce que** le dispositif comporte en outre un tube protecteur allongé (8) monté sur le manche (2) de l'autre côté dudit manche (2), ledit tube protecteur allongé (8) contenant une aiguille formant une canule (14).

2. Dispositif selon la revendication 1, **caractérisé en ce qu'**il comprend un capuchon de protection (6) monté sur le manche (2), le capuchon de protection (6) étant recouvert par le bouchon (12) et recouvrant l'aiguille rigide.

3. Dispositif selon la revendication 2, **caractérisé en ce que** le manche (2) comporte une partie avant sensiblement cylindrique (24) contenant un perçage axial recevant l'extrémité arrière de l'aiguille rigide (4), qui reçoit et maintient par serrage le capuchon (6).

4. Dispositif selon l'une quelconque des revendications 2 ou 3, **caractérisé en ce que** le capuchon (6) comporte à l'extrémité avant un bourrelet circulaire extérieur (28), présentant une face avant plane (30).

5. Dispositif selon l'une quelconque des revendications 2 à 4, caractérisé en ce le bouchon (12) recouvre le capuchon (6) et le manche (2), et présente une partie sensiblement cylindrique serrant le tube protecteur (8).

6. Dispositif selon la revendication 5, **caractérisé en ce que** les surfaces de serrage du tube protecteur (8) et du bouchon (12) l'une sur l'autre, sont formées par un moulage en matière plastique dans une partie de moule circulaire continue donnant une absence de bavure.

7. Dispositif selon l'une quelconque des revendications précédentes, **caractérisé en ce que** le tube protecteur (8) et le bouchon (12) comportent des nervures (46) ou des stries axiales (54) extérieures.

8. Dispositif selon la revendication 7, **caractérisé en ce que** le tube protecteur (8) comporte quatre nervures disposées en croix (46), qui s'étendent sensiblement sur toute la longueur du tube contenant la canule (14).

9. Dispositif selon l'une quelconque des revendications précédentes, **caractérisé en ce qu'**il comporte des nervures axiales (44, 52) assurant sur leurs contours extérieurs le serrage d'un porte-canule (10) dans le tube protecteur (8), du tube protecteur dans le manche (2), ou du manche dans le capuchon (6).

10. Dispositif selon l'une quelconque des revendications précédentes, **caractérisé en ce qu'**il comporte un porte-canule (10) présentant vers l'avant un filetage extérieur (58) comprenant deux pas différents se superposant.

11. Dispositif selon l'une quelconque des revendications précédentes, **caractérisé en ce que** ses différents composants moulés sont formés par un moulage d'une matière plastique comprenant des formes permettant un démoulage axial.

## Patentansprüche

1. Vorrichtung für ein medizinisches Verfahren, umfassend einen Griff (2), der axial an einer Seite eine starre Nadel (4) trägt, die einen Trokar bildet, der vorgesehen ist, um die Haut zu durchstechen, wobei die Vorrichtung mit einem Dichtigkeitsstopfen (12) ausgestattet ist, der mindestens die starre Nadel bedeckt, **dadurch gekennzeichnet, dass** die Vorrichtung außerdem ein verlängertes Schutzrohr (8) umfasst, das auf dem Griff (2) auf der anderen Seite des Griffs (2) montiert ist, wobei das verlängerte Schutzrohr (8) eine Nadel enthält, die eine Kanüle (14) bildet.

2. Vorrichtung nach Anspruch 1, **dadurch gekennzeichnet, dass** sie eine Schutzkappe (6) umfasst, die auf dem Griff (2) montiert ist, wobei die Schutzkappe (6) mit dem Stopfen (12) bedeckt ist und die starre Nadel bedeckt.

3. Vorrichtung nach Anspruch 2, **dadurch gekennzeichnet, dass** der Griff (2) einen im Wesentlichen zylindrischen vorderen Teil (24) umfasst, der eine axiale Bohrung enthält, die das hintere Ende der starren Nadel (4) aufnimmt, die die Kappe (6) aufnimmt und durch Klemmen festhält.

4. Vorrichtung nach einem der Ansprüche 2 oder 3, **dadurch gekennzeichnet, dass** die Kappe (6) am vorderen Ende einen äußeren runden Wulst (28) umfasst, der eine ebene vordere Seite (30) aufweist.

5. Vorrichtung nach einem der Ansprüche 2 bis 4, **dadurch gekennzeichnet, dass** der Stopfen (12) die Kappe (6) und den Griff (2) bedeckt und einen im Wesentlichen zylindrischen Teil aufweist, der das Schutzrohr (8) klemmt.

6. Vorrichtung nach Anspruch 5, **dadurch gekennzeichnet, dass** die Flächen zum Klemmen des Schutzrohrs (8) und des Stopfens (12) aufeinander durch einen Formguss aus Kunststoffmaterial in einem Teil einer kontinuierlichen runden Form gebildet sind, die zu einer Abwesenheit von Gussnähten führt.

7. Vorrichtung nach einem der vorhergehenden Ansprüche, **dadurch gekennzeichnet, dass** das Schutzrohr (8) und der Stopfen (12) äußere Rippen (46) oder axiale Rillen (54) umfassen.

8. Vorrichtung nach Anspruch 7, **dadurch gekennzeichnet, dass** das Schutzrohr (8) vier Rippen umfasst, die kreuzförmig (46) angeordnet sind, die sich im Wesentlichen auf der gesamten Länge des Rohrs, das die Kanüle (14 enthält, erstrecken.

9. Vorrichtung nach einem der vorhergehenden Ansprüche, **dadurch gekennzeichnet, dass** sie axiale Rippen (44, 52) umfasst, die auf ihren äußeren Umfängen das Klemmen eines Kanülenträgers (10) im Rohrschutz (8), des Rohrschutzes im Griff (2) oder des Griffs im Stopfen (6) sicherstellen.

10. Vorrichtung nach einem der vorhergehenden Ansprüche, **dadurch gekennzeichnet, dass** sie einen Kanülenträger (10) umfasst, der nach vorne ein Außengewinde (58) aufweist, umfassend zwei verschiedene Steigungen, die sich überlagern.

11. Vorrichtung nach einem der vorhergehenden Ansprüche, **dadurch gekennzeichnet, dass** ihre verschiedenen geformten Bestandteile durch einen Formguss eines Kunststoffmaterials erfolgt, umfassend Formen, die eine axiale Entformung ermöglichen.

## Claims

1. A device for medical procedure, comprising a handle (2) supporting axially, on one side, a rigid needle (4) forming a trocar provided to pierce the skin, the device being equipped with a seal plug (12) covering at least the rigid needle, **characterized in that** the device further includes an elongate protective tube (8) mounted on the handle (2) on the other side of said handle (2), said elongate protective tube (8) containing a needle forming a cannula (14).

2. The device according to claim 1, **characterized in that** it comprises a protective cap (6) mounted on the handle (2), the protective cap (6) being covered by the plug (12) and covering the rigid needle.

3. The device according to claim 2, **characterized in that** the handle (2) includes a substantially cylindrical front portion (24) containing an axial bore receiving the rear end of the rigid needle (4), which receives and clamps the cap (6).

4. The device according to any one of claims 2 or 3, **characterized in that** the cap (6) includes, at the front end, an outer circular bead (28) having a flat front face (30).

5. The device according to any one of claims 2 to 4, **characterized in that** the plug (12) covers the cap (6) and the handle (2), and presents a substantially cylindrical portion clamping the protective tube (8).

6. The device according to claim 5, **characterized in that** the clamping surfaces of the protective tube (8) and of the plug (12), one above the other, are formed by a plastic molding in a continuous circular mold portion giving an absence of flash.

7. The device according to any one of the preceding claims, **characterized in that** the protective tube (8) and the plug (12) include outer axial ribs (46) or ridges (54).

8. The device according to claim 7, **characterized in that** the protective tube (8) has four ribs (46) arranged in a cross-manner, which extend substantially over the entire length of the tube containing the cannula (14).

9. The device according to any one of the preceding claims, **characterized in that** it includes axial ribs (44, 52) ensuring, on their outer contours, the clamping of a cannula holder (10) in the protective tube (8), of the protective tube in the handle (2), or of the handle in the cap (6).

10. The device according to any one of the preceding claims, **characterized in that** it includes a cannula holder (10) presenting forwards an external thread (58) comprising two different superimposed threads.

11. The device according to any one of the preceding claims, **characterized in that** its different molded components are formed by a molding of a plastic material comprising shapes allowing an axial demolding.
